# EUROPEAN PATENT APPLICATION

(11) **EP 4 725 405 A1**
(43) Date of publication of application: **15.04.2026**
(21) Application number: 24819149.6
(22) Date of filing: 21.05.2024
(51) Int. Cl.: A61B 5/02

(54) **PULSE WAVE MEASUREMENT DEVICE**

(30) Priority: 06.06.2023 JP 2023093415
(71) Applicant: Minebea Mitsumi Inc., Kitasaku-gun, Nagano 3890293 (JP)
(72) Inventor: NAGAI, Takuya, Kitasaku-gun, Nagano 389-0293 (JP)
(74) Representative: Winter, Brandl - Partnerschaft mbB
(86) International application number: PCT/JP2024/018617
(87) International publication number: WO 2024/252916

(57) **Abstract**

The present pulse wave measuring device includes: cylindrical part; pulse wave sensor including housing, strain generating body on one side of housing, and facing part on the other side of housing, pulse wave sensor being held inside cylindrical part such that strain generating body is exposed from axial-direction one end of cylindrical part; cap fixed to axial-direction other end of cylindrical part; and attaching part connected to outer side of cylindrical part and attachable to a subject. First surface of facing part and second surface of cap face each other. Pivot part is provided on one of first or second surface. Recessed portion into which pivot part can be inserted is opened in the other of first and second surfaces. When pivot part and recessed portion are in contact, the pulse wave sensor can swing on a fulcrum, which is the contact between the pivot part and the recessed portion.

## Description

### TECHNICAL FIELD

The present invention relates to a pulse wave measuring device.

### BACKGROUND ART

A pulse wave measuring device including a pulse wave sensor for detecting a pulse wave generated when a heart pumps blood is known. Such a pulse wave measuring device is attachable to, for example, the wrist of a subject (for example, see PTL 1).

### CITATION LIST

### PATENT LITERATURE

PTL 1: Japanese Patent No. 5094131

### SUMMARY OF THE INVENTION

### TECHNICAL PROBLEM

Since a pulse wave measuring device using a pulse wave sensor needs to detect weak signals, in order to improve measurement accuracy, it is preferable to set the pulse wave sensor on the subject such that the detection surface of the pulse wave sensor is approximately parallel with an artery of the subject.

The present invention has been made in view of the above point, and it is an object of the present invention to provide a pulse wave measuring device that can enable the angle of a detection surface of a pulse wave sensor to follow an artery.

### SOLUTION TO THE PROBLEM

A pulse wave measuring device according to one embodiment of the present disclosure includes: a cylindrical part; a pulse wave sensor including a housing, a strain generating body provided on one side of the housing, and a facing part provided on the other side of the housing, the pulse wave sensor being held inside the cylindrical part such that the strain generating body is exposed from one end of the cylindrical part in an axial direction of the cylindrical part; a cap fixed to the other end the cylindrical part in the axial direction of the cylindrical part; and an attaching part connected to an outer side of the cylindrical part and attachable to a subject, wherein a first surface of the facing part and a second surface of the cap face each other, a pivot part is provided on one of the first surface or the second surface, a recessed portion into which the pivot part can be inserted is opened in the other of the first surface and the second surface, and when the pivot part and the recessed portion are in contact with each other, the pulse wave sensor is able to swing on a fulcrum, which is a contact part at which the pivot part and the recessed portion contact each other.

### ADVANTAGEOUS EFFECTS OF THE INVENTION

According to the disclosed technology, it is possible to provide a pulse wave measuring device that can enable the angle of a detection surface of a pulse wave sensor to follow an artery.

### BRIEF DESCRIPTION OF THE DRAWINGS

[FIG. 1] FIG. 1 is an oblique view (part 1) illustrating a pulse wave measuring device according to a first embodiment.
[FIG. 2] FIG. 2 is an oblique view (part 2) illustrating the pulse wave measuring device according to the first embodiment.
[FIG. 3] FIG. 3 is a side view illustrating the pulse wave measuring device according to the first embodiment.
[FIG. 4] FIG. 4 is an oblique view illustrating a first curved member.
[FIG. 5] FIG. 5 is an exploded oblique view illustrating a pulse wave sensor.
[FIG. 6] FIG. 6 is a view illustrating a positional relationship between a cylindrical part and a pulse wave sensor.
[FIG. 7] FIG. 7 is a view (part 1) illustrating a positional relationship between a cylindrical part, a pulse wave sensor, and a cap.
[FIG. 8] FIG. 8 is a view (part 2) illustrating a positional relationship between a cylindrical part, a pulse wave sensor, and a cap.
[FIG. 9] FIG. 9 is a view illustrating the movement of a pulse wave sensor when in contact with a subject.
[FIG. 10] FIG. 10 is a plan view illustrating a pulse wave sensor according to the first embodiment.
[FIG. 11] FIG. 11 is a cross-sectional view illustrating the pulse wave sensor according to the first embodiment.
[FIG. 12] FIG. 12 is an example of a bridge circuit.
[FIG. 13] FIG. 13 is a plan view illustrating a strain gauge according to the first embodiment.
[FIG. 14] FIG. 14 is a cross-sectional view (part 1) illustrating the strain gauge according to the first embodiment.
[FIG. 15] FIG. 15 is a cross-sectional view (part 2) illustrating the strain gauge according to the first embodiment.
[FIG. 16] FIG. 16 is a view (part 1) illustrating a biasing member.
[FIG. 17] FIG. 17 is a view (part 2) illustrating the biasing member.

### DETAILED DESCRIPTION OF THE INVENTION

Hereinafter, an embodiment for carrying out the invention will be described with reference to the drawings. In the drawings, the same components are denoted by the same reference numerals, and redundant description may be omitted.

### <First Embodiment>

### [Pulse Wave Measuring Device 1]

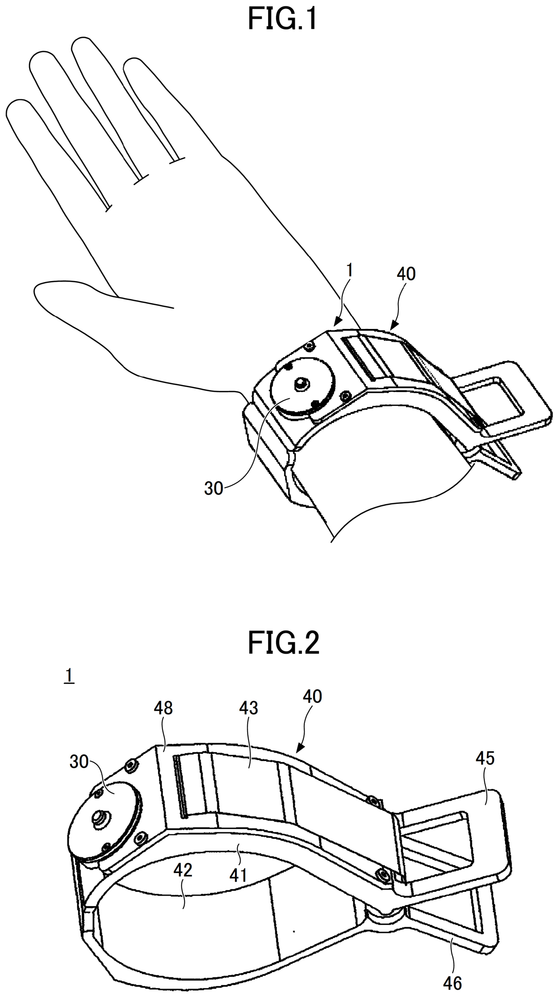

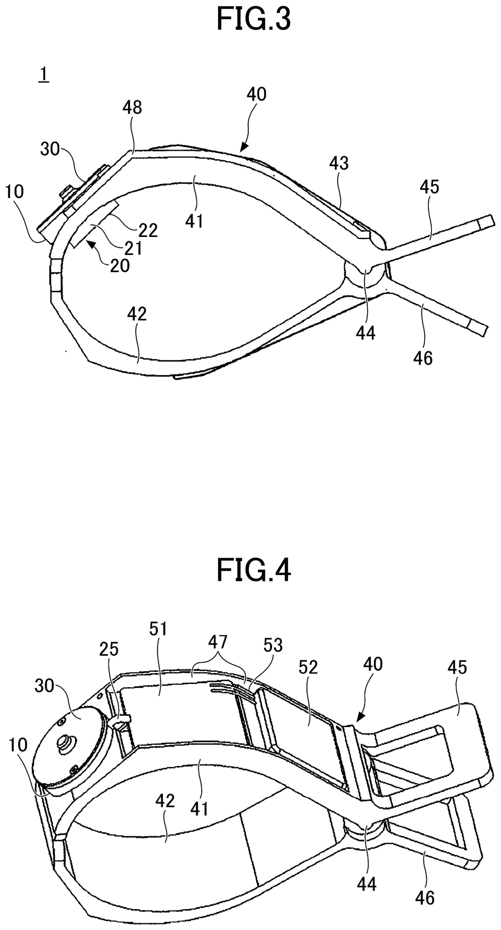

FIG. 1 is an oblique view (part 1) illustrating a pulse wave measuring device according to a first embodiment, and schematically shows a state in which the pulse wave measuring device is attached to a wrist of a subject. FIG. 2 is an oblique view (part 2) illustrating the pulse wave measuring device according to the first embodiment. FIG. 3 is a side view illustrating the pulse wave measuring device according to the first embodiment.

Referring to FIGS. 1 to 3, the pulse wave measuring device 1 is a wearable device that can be attached to a subject, and mainly includes a cylindrical part 10, a pulse wave sensor 20, a cap 30, and an attaching part 40.

The cylindrical part 10 is a member for holding the pulse wave sensor 20 thereinside. The cylindrical part 10 has, for example, a cylindrical shape with both ends open. The pulse wave sensor 20 includes a housing 21 and a strain generating body 22 provided on one side of the housing 21, and is held inside the cylindrical part 10 such that the strain generating body 22 is exposed from one end of the cylindrical part 10 in the axial direction of the cylindrical part 10. The cap 30 is fixed to the other end of the cylindrical part 10 in the axial direction of the cylindrical part 10. The attaching part 40 is connected to the outer side of the cylindrical part 10. The attaching part 40 is attachable to a subject.

The attaching part 40 includes a first curved member 41 and a second curved member 42, which are curved in opposite directions and face each other, so as to be attachable to a wrist of a subject. The first curved member 41 and the second curved member 42 are biased by a biasing member 43 bent in an approximately letter V-shape and are supported on a swing shaft 44 so as to be able to shift between a closed state and an opened state. The first curved member 41 and the second curved member 42 can be composed of, for example, resin and the like. The biasing member 43 can be composed of, for example, metal and the like. A cap member 48 is provided on the first curved member 41. The cap member 48 will be described later.

The cylindrical part 10 is situated at one end of the first curved member 41 in the longitudinal direction of the first curved member 41, and the swing shaft 44 is situated at the other end of the first curved member 41 in the longitudinal direction of the first curved member 41. The cylindrical part 10 may be formed integrally with the first curved member 41, or may be composed of a separate body that is joined to the first curved member 41. The swing shaft 44 may be formed integrally with the first curved member 41, or may be composed of a separate body that is joined to the first curved member 41.

A first operation part 45 is provided on a side of the first curved member 41 that is opposite to the cylindrical part 10 across the swing shaft 44. The first operation part 45 may be formed integrally with the first curved member 41, or may be composed of a separate body that is joined to the first curved member 41. A second operation part 46 is provided on a side of the second curved member 42 that is opposite to the cylindrical part 10 across the swing shaft 44. The second operation part 46 may be formed integrally with the second curved member 42, or may be composed of a separate body that is joined to the second curved member 42.

When attaching the pulse wave measuring device 1 to a subject, the subject or a helper or the like who helps the subject pinches the first operation part 45 and the second operation part 46 so as to bring them close to each other. Thus, the cylindrical part 10 sides of the first curved member 41 and the second curved member 42 are opened, and the pulse wave measuring device 1 can be attached to the subject.

The pulse wave measuring device 1 is attached to a wrist of the subject via the attaching part 40 such that, for example, the pulse wave sensor 20 is located close to the radial artery of the subject. A pulse wave is a waveform of a volume change of a blood vessel caused as the heart pumps out blood, and the pulse wave measuring device 1 can monitor the volume change of the blood vessel.

FIG. 4 is an oblique view for explaining the first curved member, and shows a state in which the cap member 48 is removed from the pulse wave measuring device 1. As shown in FIG. 4, the first curved member 41 may include a component installation region 47 situated between one end and the other end thereof in the longitudinal direction thereof. For example, components contributing to the detection of a pulse wave are installed in the component installation region 47. In the illustrated example, a wiring board 51 and a battery 52 are exemplified as the components contributing to the detection of a pulse wave. The wiring board 51 and the battery 52 are electrically connected via a wire 53. The pulse wave sensor 20 is electrically connected to the wiring board 51 via a wire 25. As shown in FIGS. 2 and 3, for example, the cap member 48 for protecting the components is provided on the component installation region 47.

The wiring board 51 may be mounted with, for example, an amplifier circuit for amplifying an output from a strain gauge 100, an AD converter for converting an output from the amplifier circuit into a digital signal, a signal processing semiconductor for processing a digital signal, a wireless communication semiconductor for transmitting the result of the signal processing to the outside, and the like. When connecting the pulse wave measuring device 1 to an external circuit or the like via a cable, a connector may be situated on the attaching part 40, or the cable may be extracted from the attaching part 40.

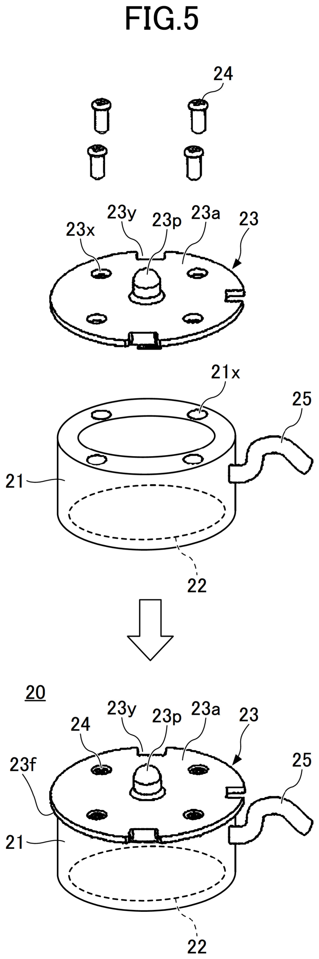

FIG. 5 is an exploded oblique view illustrating the pulse wave sensor. As shown in FIG. 5, the pulse wave sensor 20 includes, for example, a housing 21, a strain generating body 22 provided on one side of the housing 21, and a facing part 23 provided on the other side of the housing 21. The housing 21 is, for example, a cylindrical member with both ends open. The housing 21 may be composed of, for example, metal, resin, and the like.

The strain generating body 22 is approximately disk-shaped and fixed to the housing 21 by an adhesive or the like so as to close one side of the housing 21. As will be described later, the strain generating body 22 is a part on which, for example, a strain gauge is disposed to detect a pulse wave. A detailed structural example of the pulse wave sensor 20 including the strain generating body 22 will be described later.

The facing part 23 is approximately disk-shaped and fixed to the housing 21 so as to close the other side of the housing 21. The facing part 23 includes, for example, through-holes 23x and is threadedly engaged with grooves 21x provided in the housing 21 via screws 24 inserted into the through-holes 23x. The facing part 23 faces the strain generating body 22 through a space inside the housing 21. Surfaces of the facing part 23 and the strain generating body 22 facing each other are, for example, parallel.

The facing part 23 has a first surface 23a that is a surface opposite to the surface facing the strain generating body 22. The first surface 23a of the facing part 23 is, for example, a flat surface. A pivot part 23p projecting to a side opposite to the strain generating body 22 is provided in approximately the center of the first surface 23a of the facing part 23. The pivot part 23p is, for example, an approximately circular columnar member. The central axis of the pivot part 23p is, for example, perpendicular to the first surface 23a of the facing part 23.

The facing part 23 has, for example, a flange part 23f projecting to a radially outer side of the housing 21 from the outer surface of the housing 21. The flange part 23f has, for example, a ring shape. The facing part 23 may include one or a plurality of notches 23y that can be used for preventing rotation. The notches 23y are, for example, recessed from the outer peripheral side of the facing part 23 toward the center side.

The facing part 23 may be composed of, for example, metal, resin, or the like. The pivot part 23p may be integrally formed with another part of the facing part 23, or may be composed of a separate body that is joined to the another part.

The pulse wave sensor 20 may include a wire 25 for inputting and outputting electrical signals between the inside and outside of the housing 21. A plurality of mutually insulated wires may be situated inside the wire 25. One end of the plurality of wires is electrically connected to electrodes of a strain gauge described later. The pulse wave sensor 20 may include a flexible substrate or the like instead of the wire 25.

Further, one or a plurality of wiring boards may be fixed to the inner surface of the housing 21, and a pair of electrodes of the strain gauge may be electrically connected to the wiring boards via thin wires. The wiring boards may be electrically connected to the wire 25. With this structure, forces from the wire 25 are hardly transmitted to the strain generating body 22, and the detection accuracy of a pulse wave can be improved.

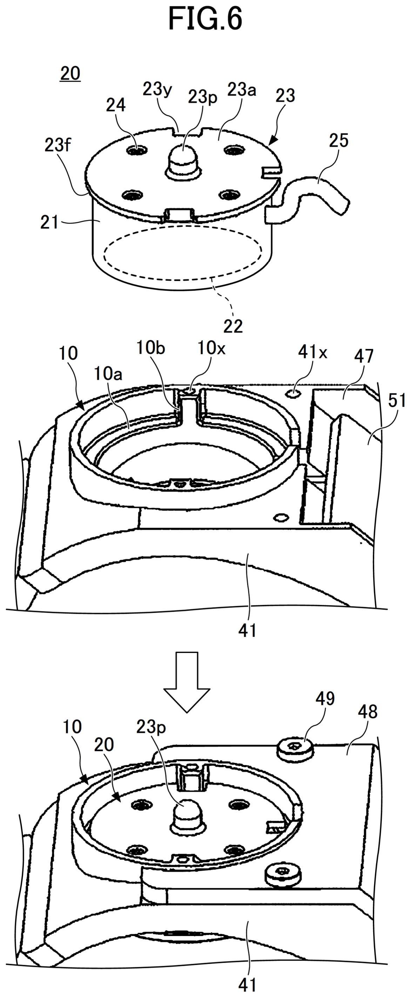

FIG. 6 is a diagram for explaining the positional relationship between the cylindrical part and the pulse wave sensor. As shown on the upper side of the arrow in FIG. 6, the inner surface of the cylindrical part 10 has, for example, a stepped surface 10a projecting to the axis side (the center side of the cylindrical part 10). The stepped surface 10a is, for example, perpendicular to the axial direction of the cylindrical part 10. The stepped surface 10a has, for example, a ring shape.

The inner surface of the cylindrical part 10 includes, for example, a positioning part 10b projecting to the axis side. The positioning part 10b is provided above the stepped surface 10a. In the illustrated example, two positioning parts 10b are provided so as to face each other. Each positioning part 10b is provided with a groove 10x.

As shown on the lower side of the arrow in FIG. 6, the pulse wave sensor 20 is held inside the cylindrical part 10 with the notches 23y aligned with the positioning parts 10b. However, the pulse wave sensor 20 is not fixed to the cylindrical part 10, and has a gap that enables the pulse wave sensor 20 to move relative to the cylindrical part 10. When the pulse wave measuring device 1 is not attached to a subject, the flange part 23f is in contact with the stepped surface 10a.

For example, after the pulse wave sensor 20 is held inside the cylindrical part 10 and the wire 25 is electrically connected to the wiring board 51, the cap member 48 may be fixed on the component installation region 47. The cap member 48 can be, for example, threadedly engaged with grooves 41x provided in the first curved member 41 via screws 49.

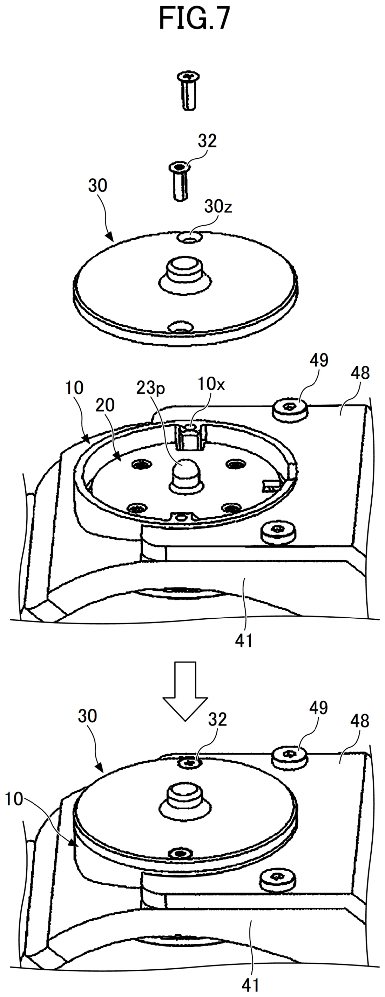

FIG. 7 is a view (part 1) for explaining the positional relationship among the cylindrical part, the pulse wave sensor, and the cap. As shown on the upper side of the arrow in FIG. 7, the cap 30 has through-holes 30z. As shown on the lower side of the arrow in FIG. 7, the cap 30 is, for example, threadedly engaged with the grooves 10x provided in the positioning parts 10b via screws 32 inserted into the through-holes 30z, and fixed to the other end of the cylindrical part 10 in the axial direction of the cylindrical part 10.

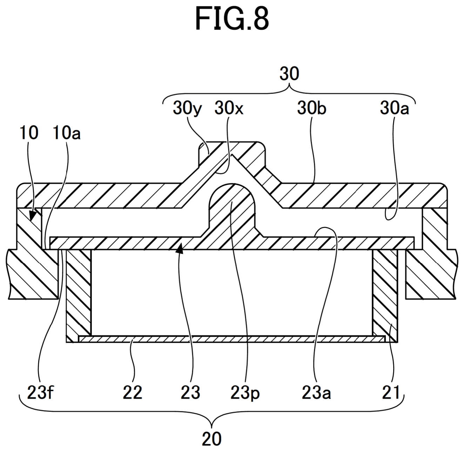

FIG. 8 is a view (part 2) for explaining the positional relationship among the cylindrical part, the pulse wave sensor, and the cap, and is a partial sectional view showing the cylindrical part 10, the pulse wave sensor 20, and the cap 30.

As shown in FIG. 8, the cap 30 has a second surface 30a and a third surface 30b opposite to the second surface 30a. The second surface 30a and the third surface 30b are, for example, parallel. A recessed portion 30x opening from the second surface 30a of the cap 30 is provided approximately in the center of the second surface 30a. The inner surface of the recessed portion 30x is, for example, one curved surface inclined from the axial direction of the cylindrical part 10. In the cross-sectional view, the inner surface of the recessed portion 30x may be partially or entirely curved.

The recessed portion 30x has, for example, a conical or frusto-conical shape. The recessed portion 30x may have a cylindrical shape having a diameter greater than that of the pivot part 23p. From the viewpoint of reducing rattling between the pivot part 23p and the recessed portion 30x to facilitate centering, it is preferable that the inner surface of the recessed portion 30x is a single curved surface, such as that of a conical shape, a frusto-conical shape, and the like, that is inclined with respect to the axial direction of the cylindrical part 10.

The cap 30 may have a projecting portion 30y projecting to the side opposite to the second surface 30a in approximately the center of the third surface 30b. The recessed portion 30x may be situated at a position coinciding with the projecting portion 30y when viewed in the axial direction of the cylindrical part 10 (vertical direction in FIG. 8), and a part of the recessed portion 30x may be situated in the projecting portion 30y. In this way, the cap 30 can be partially reduced in thickness.

The first surface 23a of the facing part 23 and the second surface 30a of the cap 30 face each other. In the facing part 23, a recessed portion 30x side of the pivot part 23p has, for example, a dome shape. Here, the dome shape is a shape in which the height from the first surface 23a is the maximum near the center axis and decreases toward the periphery. The distal end side of the pivot part 23p may be a part of a spherical surface or a part of an aspherical surface.

The flange part 23f of the facing part 23 is disposed between the stepped surface 10a of the cylindrical part 10 and the second surface 30a of the cap 30. With such a structure, the flange part 23f serves as a stopper for preventing the pulse wave sensor 20 from slipping out from the cylindrical part 10 downward.

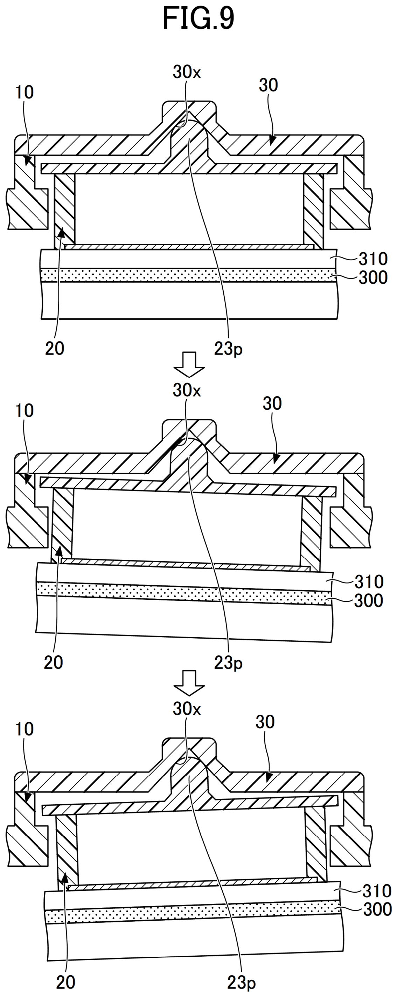

FIG. 9 is a view explaining the movement of the pulse wave sensor when in contact with a subject. In any case of FIG. 9, the attaching part 40 of the pulse wave measuring device 1 is attached to a subject. The radial artery of the subject is schematically shown at the reference numeral 300. When the attaching part 40 is attached to the subject, as shown in FIG. 9, the strain generating body 22 of the pulse wave sensor 20 is brought into contact with the skin 310 on the radial artery 300 of the subject, and the pulse wave sensor 20 is pushed up toward the cap 30, whereby the pivot part 23p and the recessed portion 30x come into contact with each other. The pivot part 23p and the recessed portion 30x come into, for example, linear contact with each other. In this way, the pivot part 23p and the recessed portion 30x can be easily centered with reduced rattling. However, this is a non-limiting feature, and the pivot part 23p and the recessed portion 30x may come into point contact or surface contact with each other.

As described above, when the attaching part 40 is not attached to the subject, the pulse wave measuring device 1 is in a first state in which the pivot part 23p and the recessed portion 30x are not in contact with each other (see FIG. 8). When the attaching part 40 is attached to the subject, it switches to a second state in which the pivot part 23p and the recessed portion 30x are in contact with each other (see FIG. 9).

As shown in the upper, middle, and lower parts of FIG. 9, when the pivot part 23p and the recessed portion 30x come into contact with each other, the pulse wave sensor 20 becomes able to swing in any of the directions defined in the range of 360 degrees, on a fulcrum, which is the contact part at which the pivot part 23p and the recessed portion 30x contact each other. That is, the detection surface of the strain generating body 22 of the pulse wave sensor 20 can be inclined at any angle along the radial artery 300 of the subject.

Thus, since the angle of the detection surface of the strain generating body 22 of the pulse wave sensor 20 can follow the radial artery 300 of the subject, the detection surface of the strain generating body 22 can contact the radial artery 300 of the subject at an appropriate angle. That is, the detection surface of the strain generating body 22 of the pulse wave sensor 20 can be placed approximately in parallel with the radial artery 300 of the subject. As a result, a weak pulse wave signal can be detected, and the pulse wave measurement accuracy can be improved.

Further, it is possible to place the pulse wave sensor 20 on the radial artery easily, by operating the first operation part 45 and the second operation part 46, to shift the pulse wave measuring device 1 between an opened state and a closed state a plurality of times and finely adjust the position of the pulse wave sensor 20. Here, it is preferable to monitor an output signal from the pulse wave sensor 20 and find a position where the amplitude of the output signal is as large as possible.

Further, in the pulse wave measuring device 1, the component installation region 47 is provided in an empty space in the first curved member 41, and components contributing to the detection of a pulse wave are installed in the component installation region 47, thereby realizing a small-sized pulse wave detecting device that is mounted with necessary components.

Further, since the pulse wave measuring device 1 is provided with the biasing member 43, it is possible to press the radial artery of the subject moderately.

### [Pulse Wave Sensor]

Here, an example of a pulse wave sensor including a plurality of strain gauges is shown as an example of the pulse wave sensor 20. Descriptions of any components of the pulse wave sensor 20 that have already been described will be omitted.

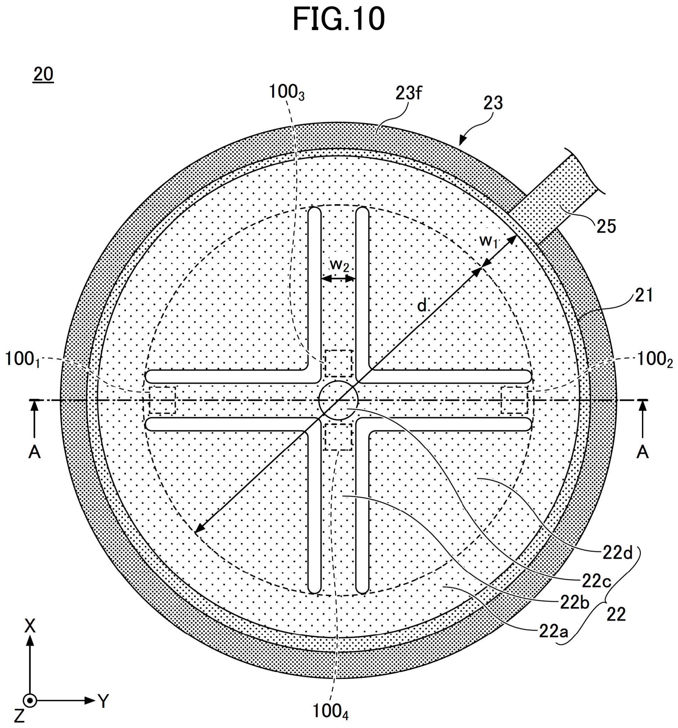

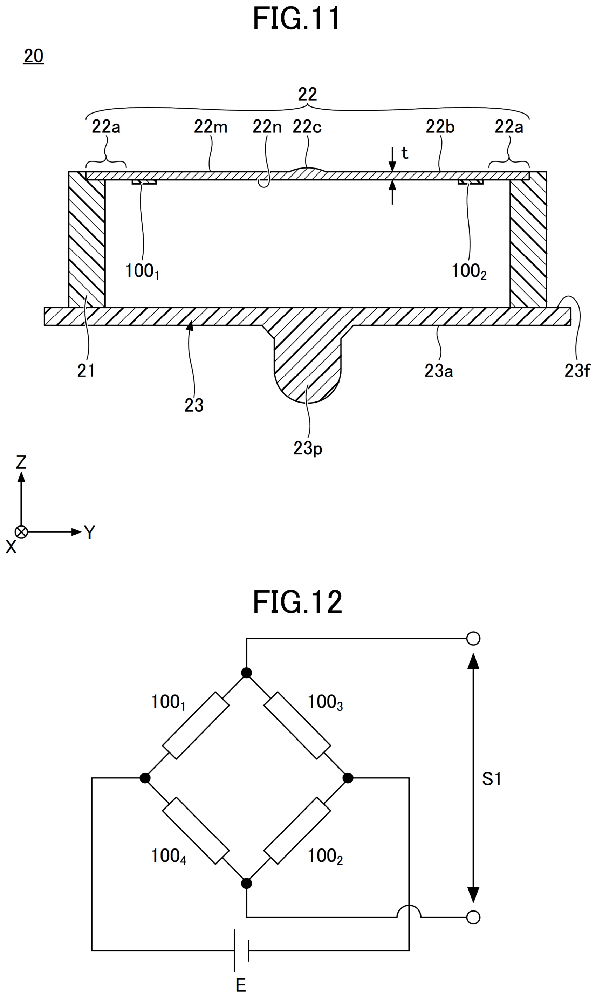

FIG. 10 is a plan view illustrating a pulse wave sensor according to the first embodiment. FIG. 11 is a cross-sectional view illustrating the pulse wave sensor according to the first embodiment, showing a cross-section along a line A-A in FIG. 10.

Referring to FIGS. 10 and 11, the pulse wave sensor 20 includes the housing 21, the strain generating body 22, the facing part 23, the wire 25, and a plurality of strain gauges (strain gauges 100₁, 100₂, 100₃, and 100₄). The facing part 23 and the wire 25 are as described above. The strain gauges 100₁, 100₂, 100₃, and 100₄ may be collectively referred to as strain gauges 100, when there is no particular need to distinguish them.

The strain generating body 22 includes a base part 22a, a beam part 22b, a load part 22c, and extension parts 22d. The strain generating body 22 has a flat plate shape. The strain generating body 22 includes a first main surface 22m and a second main surface 22n located on the side opposite to the first main surface 22m.

The material of the strain generating body 22 may be, for example, metal, ceramic, glass, and the like. Examples of the metal used as the material of the strain generating body 22 include SUS (stainless steel), copper, aluminum, and the like. The strain generating body 22 can be formed as one body by, for example, a press forming method and the like. The thickness t of the strain generating body 22 excluding the load part 22c is constant. The thickness t can be, for example, 0.03 mm or greater and 0.3 mm or less.

In the description of FIGS. 10 and 11, for convenience, a side of the pulse wave sensor 20 on which the load part 22c of the strain generating body 22 is provided is referred to as the "upper side", and a side on which the load part 22c of the strain generating body 22 is not provided is referred to as the "lower side". A surface of each part that is located on the upper side is referred to as the "upper surface", and a surface of each part that is located on the lower side is referred to as the "lower surface". However, the pulse wave sensor 20 can be used upside down. The pulse wave sensor 20 can be placed at a desirable angle. Viewing in a plan view means viewing an object in a direction normal to the first main surface 22m of the strain generating body 22 from the upper side to the lower side. A planar shape means a shape of an object when the object is viewed in the normal direction.

In the pulse wave sensor 20, the housing 21 is a part for holding the strain generating body 22. The housing 21 can be composed of, for example, metal, resin, and the like.

In the strain generating body 22, the base part 22a is a circular frame-shaped (ring-shaped) region that is on the outer side of a circular broken line shown in FIG. 10. The region that is on the inner side of the circular broken line may be referred to as a circular opening. That is, the base part 22a of the strain generating body 22 has a circular opening. The width w₁ of the base part 22a is, for example, 1 mm or greater and 5 mm or less. The inner diameter d of the base part 22a (i.e., the diameter of the circular opening) is, for example, 10 mm or greater and 15 mm or less.

The beam part 22b is provided to bridge the interior of the base part 22a. The beam part 22b includes, for example, two beams intersecting each other in a cross shape in a plan view, and the region in which the two beams intersect includes the center of the circular opening. In the example of FIG. 10, one beam that is a constituent of the cross has its longitudinal direction in the X direction, and the other beam that is a constituent of the cross has its longitudinal direction in the Y direction, and both beams cross perpendicularly to each other. It is preferable that each of the two beams crossing perpendicularly to each other is located on the inner side of the inner diameter d of the base part 22a (the diameter of the circular opening) and is as long as possible. That is, it is preferable that the length of each beam is approximately equal to the diameter of the circular opening. In each of the beams that are the constituents of the beam part 22b, the width w₂ other than in the intersecting region is constant, and is, for example, 1 mm or greater and 5 mm or less. Although it is not essential that the width w₂ is constant, it is preferable that the width w₂ is constant because a strain can be linearly detected.

The load part 22c is provided on the beam part 22b. The load part 22c is provided in, for example, the region where the two beams that are the constituents of the beam part 22b intersect. The load part 22c projects from the upper surface of the beam part 22b. The amount of projection of the load part 22c with respect to the upper surface of the beam part 22b is, for example, approximately 0.1 mm. The beam part 22b is flexible and deforms elastically when a load is applied to the load part 22c. The upper surface of the beam part 22b is a part of the first main surface 22m of the strain generating body 22.

The four extension parts 22d are fan-shaped parts extending in the direction to the beam part 22b from the inner side of the base part 22a in a plan view. A gap of approximately 1 mm is provided between the respective extension parts 22d and the beam part 22b. Since the extension parts 22d do not contribute to the sensing of the pulse wave sensor 20, they do not need to be provided.

An output signal from the pulse wave sensor 20 is generated based on outputs from the plurality of strain gauges. In the illustrated example, the pulse wave sensor 20 includes a pair of strain gauges 100₁ and 100₂ arranged to face each other across the load part 22c in a plan view on the beam extending in the Y direction on the second main surface 22n of the strain generating body 22. Further, the pulse wave sensor 20 includes another pair of strain gauges 100₃ and 100₄ arranged to face each other across the load part 22c in a plan view on the beam extending in the X direction intersecting the beam on which the pair of strain gauges 100₁ and 100₂ are arranged.

On the beam extending in the Y direction, the strain gauges 100₁ and 100₂ detect a compressive strain of the strain generating body 22 caused when the load part 22c is pressed. On the beam extending in the X direction, the strain gauges 100₃ and 100₄ detect a tensile strain of the strain generating body 22 caused when the load part 22c is pressed. The interval between the strain gauges 100₁ and 100₂ for detecting a compressive strain is wider than the interval between the strain gauges 100₃ and 100₄ for detecting a tensile strain. By arranging the strain gauges 100 in this manner, it is possible to effectively detect a compressive strain and a tensile strain and obtain a high output by means of a bridge circuit constituting a full bridge.

The strain gauges 100₁ to 100₄ are connected so as to constitute each side of one bridge circuit, and an output signal (a signal indicating a pulse wave) from the pulse wave sensor 20 can be generated by the bridge circuit. FIG. 12 is an example of a bridge circuit. In the bridge circuit shown in FIG. 12, the strain gauge 100₁ constitutes one side on the upper left. The strain gauge 100₂ constitutes one side on the lower right. The strain gauge 100₃ constitutes one side on the upper right. The strain gauge 100₄ constitutes one side on the lower left.

In FIG. 12, a DC voltage E is supplied between a connecting part at which the upper left side and the lower left side are connected and a connecting part at which the upper right side and the lower right side are connected. Thus, an analog voltage output signal S1 can be obtained from between a connecting part at which the upper left side and the upper right side are connected and a connecting part at which the lower left side and the lower right side are connected. For example, the wiring pattern constituting the bridge circuit can be provided on the wiring board 51.

In the pulse wave sensor 20, when the load part 22c contacts the radial artery of the subject, a load is applied to the load part 22c in accordance with a pulse wave of the subject, and the beam part 22b elastically deforms, thereby changing the resistance values of the resistors of the strain gauges 100. The pulse wave sensor 20 can detect the pulse wave based on the changes in the resistance values of the resistors of the strain gauges 100 caused by the deformation of the beam part 22b. The pulse wave is detected from the bridge circuit in the form of the output signal S1 as a periodic voltage change.

In the foregoing description, an example in which the pulse wave sensor 20 includes four strain gauges and generates the output signal S1 by connecting the four strain gauges as a full bridge has been shown. However, the pulse wave sensor 20 may include two strain gauges and generate the output signal S1 by connecting the two strain gauges as a half bridge.

### [Strain Gauge 100]

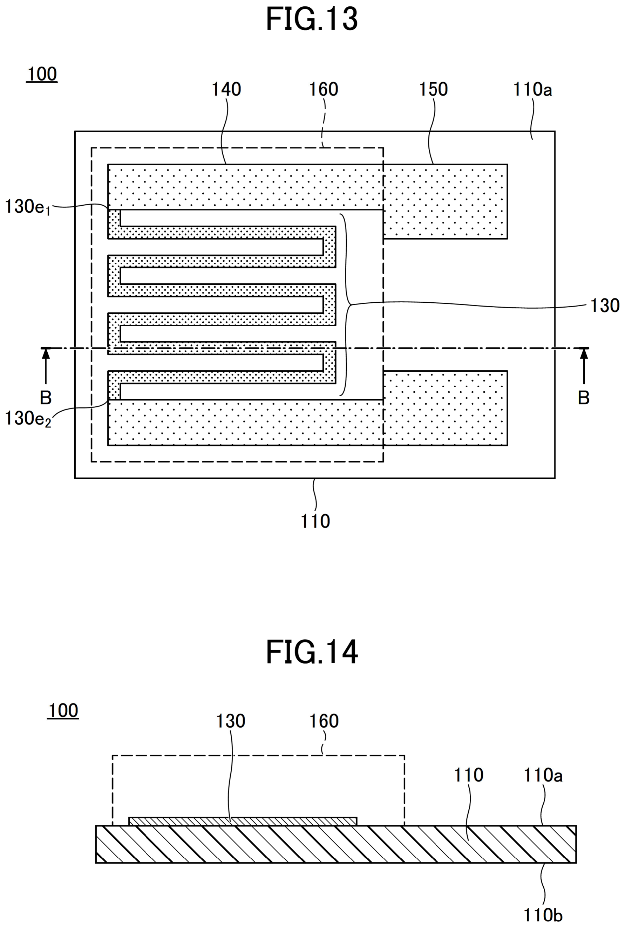

FIG. 13 is a plan view illustrating a strain gauge according to the first embodiment. FIG. 14 is a cross-sectional view (part 1) illustrating the strain gauge according to the first embodiment, and shows a cross section along a line B-B in FIG. 13.

Referring to FIGS. 13 and 14, the strain gauge 100 includes a substrate 110, a resistor 130, a wiring 140, electrodes 150, and a cover layer 160. That is, the strain gauge 100 includes the resistor 130 as a detection element. The cover layer 160 can be provided on an as-needed basis. In FIGS. 13 and 14, only the outer edges of the cover layer 160 are shown by broken lines for convenience. Each component of the strain gauge 100 will be described in detail.

In the description of the strain gauge with reference to FIGS. 13 to 15, the definitions of the upper and lower surfaces are different from those in the other drawings. Specifically, in FIGS. 13 to 15, a side of the strain gauge 100 on which the resistor 130 of the substrate 110 is provided is referred to as the "upper side" and a side of the strain gauge 100 on which the resistor 130 is not provided is referred to as the "lower side" for convenience. Further, a surface of each part located on the upper side is referred to as the "upper surface" and a surface of each part located on the lower side is referred to as the "lower surface". However, the strain gauge 100 can be used upside down. The strain gauge 100 can also be placed at a desirable angle. Viewing in a plan view means viewing an object in a direction normal to the upper surface 110a of the substrate 110 from the upper side to the lower side. A planar shape means the shape of the object when viewed in the normal direction. The strain gauge 100 is attached to the second main surface 22n of the strain generating body 22 such that the substrate 110 faces the second main surface 22n of the strain generating body 22.

The substrate 110 is a member serving as a base layer on which the resistor 130 and the like are formed. The substrate 110 has flexibility. The thickness of the substrate 110 is not particularly limited and may be appropriately determined in accordance with the purpose of use of the strain gauge 100 and the like. For example, the thickness of the substrate 110 may be approximately 5 µm to 500 µm. The thickness of the substrate 110 is preferably within the range of 5 µm to 200 µm from the viewpoint of the transmissibility of a strain from the second main surface 22n of the strain-causing member 22 to the sensitive part and dimensional stability against environmental changes. The thickness of the substrate 110 is preferably 10 µm or greater from the viewpoint of insulation performance.

The substrate 110 is formed from, for example, an insulating resin film, such as a polyimide (PI) resin, an epoxy resin, a polyetheretherketone (PEEK) resin, a polyethylene naphthalate (PEN) resin, a polyethylene terephthalate (PET) resin, a polyphenylene sulfide (PPS) resin, a liquid crystal polymer (LCP) resin, a polyolefin resin, and the like. The film refers to a member having a thickness of approximately 500 µm or less and having flexibility.

When the substrate 110 is formed from an insulating resin film, the insulating resin film may contain fillers, impurities, or the like. The substrate 110 may be formed from, for example, an insulating resin film containing a filler, such as silica, alumina, or the like.

Examples of the material of the substrate 110 other than resin include crystalline materials, such as SiO₂, ZrO₂ (including YSZ), Si, Si₂N₃, Al₂O₃ (including sapphire), ZnO, perovskite ceramics (CaTiO₃, BaTiO₃), and the like. In addition to the aforementioned crystalline materials, amorphous glass or the like may be used as the material of the substrate 110. As the material of the substrate 110, a metal such as aluminum, aluminum alloy (duralumin), titanium, and the like may be used. When a metal substrate 110 is used, an insulating film is provided so as to cover the upper surface 110a.

The resistor 130 is a thin film formed in a predetermined pattern on the upper side of the substrate 110. In the strain gauge 100, the resistor 130 is a sensitive part that generates a resistance change upon receiving a strain. The resistor 130 may be directly formed on the upper surface 110a of the substrate 110, or may be formed on the upper surface 110a of the substrate 110 via another layer. In FIG. 13, for convenience, the resistor 130 is shown with a dense stippled pattern.

The resistor 130 has a structure in which a plurality of thin and long portions are arranged at predetermined intervals with their longitudinal direction set in the same direction (the direction of the B-B line in the example of FIG. 13), and the end portions of adjacent long and thin portions are connected on alternate sides, to have a zigzag-folded-back shape as a whole. The longitudinal direction of the plurality of thin and long portions is a grid direction, and the direction perpendicular to the grid direction is a grid width direction (the direction perpendicular to the line B-B in the example of FIG. 13).

End portions, on one side in the longitudinal direction, of two thin and long portions positioned on the outermost sides in the grid width direction are bent in the grid width direction to form respective terminals 130e₁ and 130e₂ of the resistor 130 in the grid width direction. The respective terminals 130e₁ and 130e₂ of the resistor 130 in the grid width direction are electrically connected to the electrodes 150 via the wiring 140. In other words, the wiring 140 electrically connects the terminals 130e₁ and 130e₂ of the resistor 130 in the grid width direction to the electrodes 150, respectively.

The resistor 130 can be formed, for example, from a material containing chromium (Cr), a material containing nickel (Ni), or a material containing both Cr and Ni. That is, the resistor 130 can be formed from a material containing at least one of Cr or Ni. Examples of the material containing Cr include a Cr mixed phase film. Examples of the material containing Ni include copper nickel (Cu-Ni). Examples of the material containing both Cr and Ni include nickel chromium (Ni-Cr).

Here, the Cr mixed phase film is a film in which Cr, CrN, Cr₂N and the like are mixed. The Cr mixed phase film may contain unavoidable impurities, such as chromium oxide and the like.

The thickness of the resistor 130 is not particularly limited, and can be appropriately determined in accordance with the purpose of use of the strain gauge 100 and the like. For example, the thickness of the resistor 130 may be approximately 0.05 µm to 2 µm. In particular, when the thickness of the resistor 130 is 0.1 µm or greater, the crystallinity of the crystals constituting the resistor 130 (for example, the crystallinity of α-Cr) is improved. In addition, when the thickness of the resistor 130 is 1 µm or less, (i) cracks in the film and (ii) warpage of the film from the substrate 110, which are caused by the internal stress in the film constituting the resistor 130 are reduced.

Taking into consideration making lateral sensitivity less likely to occur and the wire breakage countermeasures, the width of the resistor 130 is preferably 10 µm or greater and 100 µm or less. To be more specific, the width of the resistor 130 is preferably 10 µm or greater and 70 µm or less, and more preferably 10 µm or greater and 50 µm or less.

For example, when the resistor 130 is a Cr mixed phase film, the stability of the gauge characteristics can be improved by using alpha chromium (α-Cr), which is a stable crystal phase, as the main component. Moreover, for example, when the resistor 130 is a Cr mixed phase film, using α-Cr as the main component of the resistor 130 can bring the gauge factor of the strain gauge 100 to 10 or more, and the temperature coefficient of gauge factor TCS and the temperature coefficient of resistance TCR to within a range of -1,000 ppm/°C to +1,000 ppm/°C. Here, the term "main component" means that the component occupies 50% by weight or more of all the materials constituting the resistor. From the viewpoint of improving the gauge characteristics, the resistor 130 preferably contains 80% by weight or more of α-Cr. Moreover, from the same viewpoint, the resistor 130 more preferably contains 90% by weight or more of α-Cr. Here, α-Cr is Cr having a bcc structure (body-centered cubic lattice structure).

When the resistor 130 is a Cr mixed phase film, CrN and Cr₂N contained in the Cr mixed phase film are preferably 20% by weight or less. When CrN and Cr₂N contained in the Cr mixed phase film are 20% by weight or less, the lowering of the gauge factor of the strain gauge 100 can be suppressed.

As the ratio between CrN and Cr₂N in the Cr mixed phase film, the proportion of Cr₂N is preferably 80% by weight or more and less than 90% by weight relative to the total weight of CrN and Cr₂N. More specifically, as the ratio, the proportion of Cr₂N is more preferably 90% by weight or more and less than 95% by weight relative to the total weight of CrN and Cr₂N. Cr₂N has semiconducting properties. Therefore, when the proportion of Cr₂N is 90% by weight or more and less than 95% by weight, the decrease in TCR (negative TCR) becomes more remarkable. Moreover, when the proportion of Cr₂N mentioned above is 90% by weight or more and less than 95% by weight, ceramization of the resistor 130 can be reduced. Therefore, it is possible to make brittle fracture of the resistor 130 less likely to occur.

Meanwhile, CrN has an advantage of being chemically stable. Including more CrN in the Cr mixed phase film can reduce the possibility of occurrence of unstable N. Therefore, a stable strain gauge can be obtained. Here, the term "unstable N" means trace N₂ or atomic N that may be present in the Cr mixed phase film. These types of unstable N may escape the film depending on the external environment (for example, a high-temperature environment). When unstable N escapes the film, the film stress in the Cr mixed phase film may change.

When the Cr mixed phase film is used as the material of the resistor 130 in the strain gauge 100, it is possible to achieve high sensitivity and size reduction. For example, while the output from existing strain gauges is approximately 0.04 mV/2 V, an output of 0.3 mV/2 V or greater can be obtained when the Cr mixed phase film is used as the material of the resistor 130. Further, while the size (gauge length × gauge width) of existing strain gauges is approximately 3 mm × 3 mm, the size (gauge length × gauge width) can be reduced to approximately 0.3 mm × 0.3 mm when the Cr mixed phase film is used as the material of the resistor 130.

The wiring 140 is provided on the substrate 110. The wiring 140 is electrically connected to the resistor 130 and the electrodes 150. The wiring 140 may be patterned in any shape, and is not limited to a straight line. The wiring 140 can have any width and any length. In FIG. 13, for convenience, the wiring 140 is shown with a stippled pattern that is less dense than that of the resistor 130.

The electrodes 150 are provided on the substrate 110. The electrodes 150 are electrically connected to the resistor 130 via the wiring 140. The electrodes 150 are formed in an approximately rectangular shape having a wider width than that of the wiring 140 in a plan view. The electrodes 150 are a pair of electrodes for outputting changes in the resistance value of the resistor 130 caused by a strain to the outside. For example, lead wires for external connection or the like are joined to the electrodes 150. A metal layer having a low resistance, such as copper and the like, or a metal layer having good solderability, such as gold and the like, may be laminated on the upper surfaces of the electrodes 150. Although the resistor 130, the wiring 140, and the electrodes 150 are designated by different reference numerals for convenience, they can be composed of the same material in the same process as an integrated body. In FIG. 13, for convenience, the electrodes 150 are shown with a stippled pattern having the same density as that of the wiring 140.

The cover layer 160 (protective layer) is provided as needed on the upper surface 110a of the substrate 110 so as to cover the resistor 130 and the wiring 140 and expose the electrodes 150. Examples of the material of the cover layer 160 include insulating resins, such as a PI resin, an epoxy resin, a PEEK resin, a PEN resin, a PET resin, a PPS resin, a composite resin (for example, a silicone resin and a polyolefin resin), and the like. The cover layer 160 may contain a filler and a pigment. The thickness of the cover layer 160 is not particularly limited and may be appropriately selected in accordance with the purpose. For example, the thickness of the cover layer 160 may be approximately 2 µm to 30 µm. By providing the cover layer 160, it is possible to inhibit mechanical damage to the resistor 130. Further, by providing the cover layer 160, it is possible to protect the resistor 130 from moisture and the like.

### [Method of Manufacturing Strain Gauge 100]

In the strain gauge 100 according to the present embodiment, the resistor 130, the wiring 140, the electrodes 150, and the cover layer 160 are formed on the substrate 110. Another layer (e.g., a functional layer described later) may be formed between the substrate 110 and the layers of these members.

Hereinafter, a method of manufacturing the strain gauge 100 will be described. In order to manufacture the strain gauge 100, first, the substrate 110 is prepared, and a metal layer (referred to as a metal layer A for convenience) is formed on the upper surface 110a of the substrate 110. The metal layer A is a layer that is ultimately patterned to become the resistor 130, the wiring 140, and the electrodes 150. Therefore, the material and thickness of the metal layer A are the same as the material and thickness of the resistor 130, the wiring 140, and the electrodes 150.

The metal layer A can be formed by, for example, a magnetron sputtering method using, as a target, a raw material from which the metal layer A can be formed. Instead of the magnetron sputtering method, the metal layer A may be formed by a reactive sputtering method, a vapor deposition method, an arc ion plating method, a pulsed laser deposition method, and the like. After the metal layer A is formed on the upper surface 110a of the substrate 110, the metal layer A is patterned into planar shapes that are the same as that of the resistor 130, the wiring 140, and the electrodes 150 of FIG. 13, by a well-known photolithography method.

The metal layer A may be formed after an underlying layer is formed on the upper surface 110a of the substrate 110. For example, a functional layer having a predetermined thickness may be deposited in vacuum on the upper surface 110a of the substrate 110 by a conventional sputtering method. By providing the underlying layer in this way, it is possible to stabilize the gauge characteristics of the strain gauge 100.

In the present application, the functional layer refers to a layer having a function of promoting the crystal growth of at least the metal layer A (resistor 130) to be deposited thereon. It is preferable that the functional layer further has a function of preventing oxidation of the metal layer A due to oxygen or moisture contained in the substrate 110 and/or a function of improving adhesiveness between the substrate 110 and the metal layer A. The functional layer may further have other functions.

The insulating resin film constituting the substrate 110 might contain oxygen or moisture, and Cr may form an autoxidized film. Therefore, especially when the metal layer A contains Cr, it is preferable to form a functional layer having a function of preventing oxidation of the metal layer A.

Thus, by providing a functional layer under the metal layer A, it is possible to promote the crystal growth of the metal layer A, and to form the metal layer A composed of stable crystal phases. As a result, the stability of the gauge characteristics of the strain gauge 100 is improved. In addition, diffusion of a material that is a constituent of the functional layer into the metal layer A improves the gauge characteristics of the strain gauge 100.

Examples of the material of the functional layer include one or more types of metals selected from the group consisting of chromium (Cr), titanium (Ti), vanadium (V), niobium (Nb), tantalum (Ta), nickel (Ni), yttrium (Y), zirconium (Z), hafnium (Hf), silicon (Si), carbon (C), zinc (Zn), copper (Cu), bismuth (Bi), iron (Fe), molybdenum (Mo), tungsten (W), ruthenium (R), rhodium (Rh), rhenium (Re), osmium (Os), iridium (Ir), platinum (Pt), palladium (Pd), silver (Ag), gold (Au), cobalt (Co), manganese (Mn), and aluminum (Al), alloys of any of the metals in this group, or compounds of any of the metals in this group.

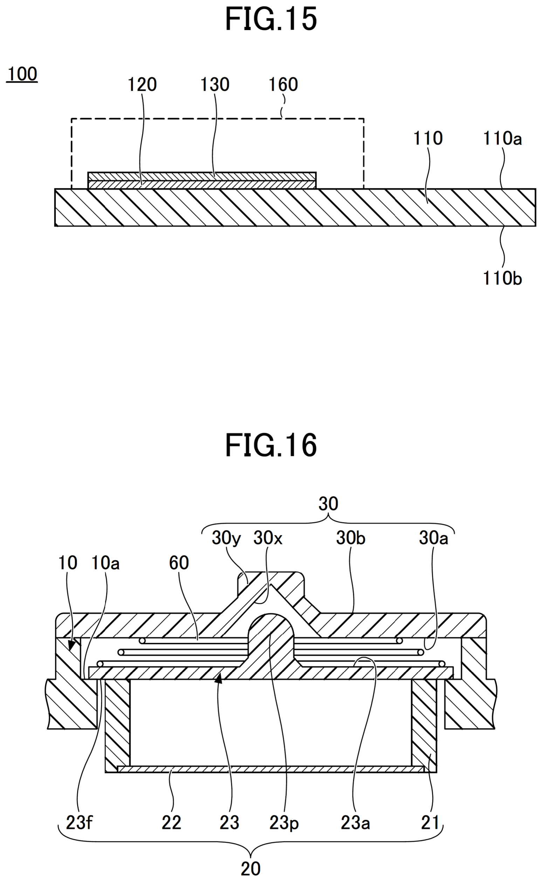

FIG. 15 is a cross-sectional view (part 2) illustrating the strain gauge according to the first embodiment. FIG. 15 shows a cross-sectional shape of the strain gauge 100 when a functional layer 120 is provided as an underlying layer of the resistor 130, the wiring 140, and the electrodes 150.

For example, the planar shape of the functional layer 120 may be patterned to be approximately the same as the planar shapes of the resistor 130, the wiring 140, and the electrodes 150. However, the planar shape of the functional layer 120, and that of the resistor 130, the wiring 140, and the electrodes 150 do not need to be approximately the same. For example, when forming the functional layer 120 from an insulating material, the functional layer 120 may be patterned to have a shape different from the planar shapes of the resistor 130, the wiring 140, and the electrodes 150. In this case, for example, the functional layer 120 may be formed solidly on the region where the resistor 130, the wiring 140, and the electrodes 150 are to be formed. Alternatively, the functional layer 120 may be formed solidly on the entirety of the upper surface of the substrate 110.

After forming the resistor 130, the wiring 140, and the electrodes 150, a cover layer 160 is formed on the upper surface 110a of the substrate 110, as needed. The cover layer 160 covers the resistor 130 and the wiring 140, but the electrodes 150 may be exposed from the cover layer 160. For example, the cover layer 160 can be formed by laminating a semicured thermosetting insulating resin film on the upper surface 110a of the substrate 110 so as to cover the resistor 130 and the wiring 140 and expose the electrodes 150, and then heating and curing the insulating resin film. The strain gauge 100 is completed through the above steps.

### <First Modification of First Embodiment>

In the first modification of the first embodiment, an example in which a biasing member is disposed between the pulse wave sensor and the cap will be shown. In the first modification of the first embodiment, description of the same components as those of the embodiment already described may be omitted.

FIG. 16 is a view (part 1) explaining the biasing member, and is a partial sectional view showing the cylindrical part 10, the pulse wave sensor 20, and the cap 30. The structure of FIG. 16 is different from the structure shown in FIG. 8 in that a biasing member 60 is disposed inside the cylindrical part 10 between the first surface 23a of the facing part 23 and the second surface 30a of the cap 30.

The biasing member 60 can be disposed in contact with the first surface 23a of the facing part 23 and the second surface 30a of the cap 30. By disposing the biasing member 60, it is possible to bias the pulse wave sensor 20 in a direction to be away from the second surface 30a of the cap 30.

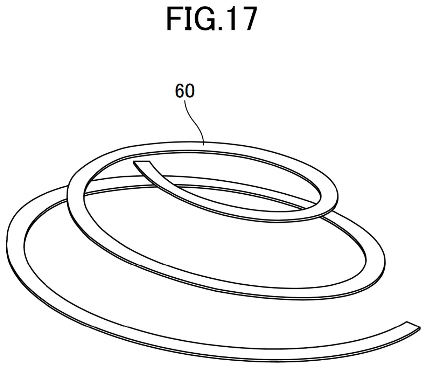

FIG. 17 is a view (part 2) explaining the biasing member, and is an oblique view showing only the biasing member. As shown in FIG. 17, the biasing member 60 is, for example, a helical (spiral) leaf spring. The biasing member 60 can be disposed such that the pivot part 23p is approximately in the center thereof in a plan view. The biasing member 60 can be composed of, for example, a metal, a resin, a rubber, and the like.

The biasing member 60 may be a conical spring or a cylindrical spring. When the biasing member is a conical spring, the height thereof in the compressed state can be less than that of a cylindrical spring. Therefore, the height of the cylindrical part 10 can be reduced. When the biasing member 60 is a conical spring, in order to stably dispose the biasing member 60, it is preferable to dispose it such that a part of the conical spring that has a small diameter is on the cap 30 side.

As long as the pulse wave sensor 20 can be biased in a direction to be away from the second surface 30a of the cap 30, the shape and material of the biasing member 60 do not matter.

By disposing the biasing member 60 in this way, it is possible to maintain the flange part 23f in contact with the stepped surface 10a even when vibration or the like is applied to the pulse wave sensor 20 in a state in which the pulse wave sensor is not in contact with a wrist or the like of a subject. Therefore, it is possible to prevent rattling between the pulse wave sensor 20 and the cap 30. Thus, for example, it is possible to reduce the possibility that an abnormal sound occurs when the pulse wave measuring device 1 is carried, and the like.

Although preferable embodiments and the like have been described in detail above, the embodiments and the like described above are non-limiting, and various modifications and replacements are applicable to the embodiments and the like described above without departing from the scope described in the claims.

For example, in the above embodiment, the pivot part 23p is provided on the first surface 23a of the facing part 23, and the recessed portion 30x into which the pivot part 23p can be inserted is opened in the second surface 30a of the cap 30. However, the present invention is not limited to this, and the pivot part may be provided on the second surface 30a of the cap 30, and the recessed portion into which the pivot part can be inserted may be opened in the first surface 23a of the facing part 23. That is, the pivot part may be provided on one of the first surface 23a of the facing part 23 or the second surface 30a of the cap 30, and the recessed portion into which the pivot part can be inserted may be opened in the other of the first surface 23a and the second surface 30a.

The attaching part 40 may have a wristwatch strap shape and the like instead of a clip shape shown in FIG. 3 and the like. For example, the first curved member 41 and the second curved member 42 may be composed of leather, rubber, and the like, and the ends of the first curved member 41 and the second curved member 42 may be detachably connected by a hook-and-loop fastener and the like. Alternatively, the first curved member 41 and the second curved member 42 may be integrally formed into a single strap shape and composed of a stretchable material.

The structure of the pulse wave sensor 20 is not limited to that shown in FIG. 10 and the like, and may be any structure. For example, a structure in which no slit is provided around the beam part may be used. A side of the strain generating body that faces the first surface may be coated with a resin and the like.

The present international application claims priority to Japanese Patent Application No. 2023-093415 filed on June 6, 2023, and the entire contents of Japanese Patent Application No. 2023-093415 are incorporated herein.

### REFERENCE SIGNS LIST

1: pulse wave measuring device, 10: cylindrical part, 10a: stepped surface, 10b: positioning part, 10x: groove, 20: pulse wave sensor, 21: housing, 21x: groove, 22: strain generating body, 22a: base part, 22b: beam part, 22c: load part, 22d: extension part, 22 m: first main surface, 22n: second main surface, 23: facing part, 23a: first surface, 23f: flange part, 23p: pivot part, 23x: through-hole, 23y: notch, 24: screw, 25: wire, 30: cap, 30a: second surface, 30b: third surface, 30x: recessed portion, 30y: projecting portion, 30z: through-hole, 32: screw, 40: attaching part, 41: first curved member, 41x: groove, 42: second curved member, 43: biasing member, 44: swing shaft, 45: first operation part, 46: second operation part, 47: component installation region, 48: cap member, 49: screw, 51: wiring board, 52: battery, 53: wire, 60: biasing member, 100₁, 100₂, 100₃, 100₄: strain gauge, 110: substrate, 110a: upper surface, 130: resistor, 130e₁, 130e₂: terminal, 140: wiring, 150: electrode, 160: cover layer, 300: radial artery, 310: skin

## Claims

1. A pulse wave measuring device, comprising:
a cylindrical part;
a pulse wave sensor including a housing, a strain generating body provided on one side of the housing, and a facing part provided on the other side of the housing, the pulse wave sensor being held inside the cylindrical part such that the strain generating body is exposed from one end of the cylindrical part in an axial direction of the cylindrical part;
a cap fixed to the other end of the cylindrical part in the axial direction of the cylindrical part; and
an attaching part connected to an outer side of the cylindrical part and attachable to a subject,
wherein a first surface of the facing part and a second surface of the cap face each other,
a pivot part is provided on one of the first surface or the second surface, and a recessed portion into which the pivot part can be inserted is opened in the other of the first surface and the second surface, and
when the pivot part and the recessed portion are in contact with each other, the pulse wave sensor is able to swing on a fulcrum, which is a contact part at which the pivot part and the recessed portion contact each other.

2. The pulse wave measuring device according to claim 1,
wherein when the attaching part is not attached to the subject, the pulse wave measuring device is in a first state in which the pivot part and the recessed portion are not in contact with each other, and
when the attaching part is attached to the subject, the pulse wave measuring device switches to a second state in which the pivot part and the recessed portion are in contact with each other.

3. The pulse wave measuring device according to claim 2,
wherein in the second state, the pivot part and the recessed portion are in linear contact with each other.

4. The pulse wave measuring device according to any one of claims 1 to 3,
wherein a side of the pivot part that faces the recessed portion has a dome shape, and
an inner surface of the recessed portion is one curved surface inclined with respect to the axial direction of the cylindrical part.

5. The pulse wave measuring device according to claim 4,
wherein the recessed portion has a conical shape or a frusto-conical shape.

6. The pulse wave measuring device according to any one of claims 1 to 5,
wherein the facing part includes a flange part projecting to a radially outer side of the housing from an outer surface of the housing,
an inner surface of the cylindrical part has a stepped surface projecting axially inward,
the flange part is disposed between the stepped surface and the second surface, and
the flange part serves as a stopper configured to prevent the pulse wave sensor from slipping out from the cylindrical part.

7. The pulse wave measuring device according to any one of claims 1 to 6,
wherein the cap has a projecting portion on a third surface opposite to the second surface,
the recessed portion is situated at a position coinciding with the projecting portion when viewed in the axial direction of the cylindrical part, and
a part of the recessed portion is situated in the projecting portion.

8. The pulse wave measuring device according to any one of claims 1 to 7,
wherein a first biasing member configured to bias the pulse wave sensor in a direction away from the second surface is disposed between the first surface and the second surface.

9. The pulse wave measuring device according to any one of claims 1 to 8,
wherein the strain generating body includes a plurality of strain gauges, and
the pulse wave sensor detects a pulse wave based on changes in resistance values of resistors of the plurality of strain gauges.

10. The pulse wave measuring device according to claim 9,
wherein the plurality of strain gauges include a pair of strain gauges configured to detect a compressive strain of the strain generating body and another pair of strain gauges configured to detect a tensile strain of the strain generating body,
the pair of strain gauges and the another pair of strain gauges are connected so as to form respective sides of one bridge circuit, and
a signal indicative of the pulse wave is generated by the bridge circuit.

11. The pulse wave measuring device according to any one of claims 1 to 10,
wherein the attaching part includes a first curved member and a second curved member that are curved in opposite directions and face each other so as to be attachable to a wrist of the subject,
the first curved member and the second curved member are biased by a second biasing member, and are supported on a swing shaft so as to be able to shift between a closed state and an opened state,
the cylindrical part is situated at one end of the first curved member in a longitudinal direction of the first curved member, and
the swing shaft is situated at the other end of the first curved member in the longitudinal direction of the first curved member.

12. The pulse wave measuring device according to claim 11,
wherein the first curved member includes a component installation region situated between the one end and the other end thereof in the longitudinal direction thereof, and
a component contributing to detection of a pulse wave is installed in the component installation region.
